(19)

Europäisches Patentamt
European Patent Office
Office européen des brevets

(11) **EP 4 375 253 A1**

(12) **EUROPEAN PATENT APPLICATION**

(43) Date of publication:
**29.05.2024 Bulletin 2024/22**

(21) Application number: **23200245.1**

(22) Date of filing: **27.09.2023**

(51) International Patent Classification (IPC):
$C03C\ 3/097$ (2006.01)  $C03C\ 4/00$ (2006.01)
$C03C\ 10/00$ (2006.01)

(52) Cooperative Patent Classification (CPC):
**C03C 4/0021; C03C 3/097; C03C 10/0027;**
C03C 10/0009

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.10.2022 CN 202211309304**

(71) Applicant: **Shenzen Yurucheng Dental Materials
Co., Ltd.
Shenzhen, Pingshan 518000 (CN)**

(72) Inventors:
• **LI, Zongyu
  Shenzhen, 518000 (CN)**
• **LIU, Wei
  Shenzhen, 518000 (CN)**
• **WANG, Jian
  Shenzhen, 518000 (CN)**
• **LIU, Jianjun
  Shenzhen, 518000 (CN)**
• **ZHANG, Yu
  Shenzhen, 518000 (CN)**

(74) Representative: **Monteiro Alves, Inês
  Alameda Dos Oceanos, № 41K-21
  Parque das Nações
  1990-207 Lisboa (PT)**

(54) **GLASS CERAMIC COMPOSITION, PREPARATION METHOD AND APPLICATION THEREOF**

(57)    Disclosed are a glass ceramic composition, a preparation method and application thereof. The glass ceramic composition according to the present application includes: 58% to 72% of $SiO_2$; 0% to 4% of $Al_2O_3$; 0% to 5% of $Na_2O$; 3% to 8% of $K_2O$; 8% to 17% of $Li_2O$; 2.5% to 5% of $P_2O_5$, 0% to 2% of MgO, 0% to 2.5% of $B_2O_3$; 0% to 5% of ZnO; and 0% to 3% of $ZrO_2$. It is possible to prepare such lithium metasilicate glass ceramic with a crystal phase of lithium metasilicate, a crystallinity of greater than 50%, a grain size of 30nm to 60nm and a hardness of 500kgf/mm$^2$ to 590 kgf/mm$^2$. The composition does not require an introduction of a high proportion of zirconia content, and can be ground to a thickness of 0.2mm without edge collapse.

FIG. 2

EP 4 375 253 A1

**Description**

**TECHNICAL FIELD**

The present application relates to the technical field of dental repair materials, and in particular, to a glass ceramic composition, a preparation method and an application thereof.

**BACKGROUND**

Lithium disilicate glass ceramics are widely used in the field of dental restoration due to their excellent mechanical properties and outstanding aesthetic effects. At present, it is mainly applied to veneers, dental bridges, crowns, inlays, implant abutments, etc. Due to the high strength of the lithium disilicate glass ceramics, it is difficult to process and grind them by using computer aided design (CAD)/computer aided manufacturing (CAM), which seriously affects the service life of the bur. In the industry, a matrix glass is often crystallized to generate a lithium metasilicate glass ceramic, then is molded and processed, finally the formed lithium metasilicate glass ceramic is heat treated to obtain a lithium disilicate glass ceramic. Since the strength of the lithium metasilicate glass ceramic is lower than that of the lithium disilicate glass ceramic, molding and processing are relatively easy.

In actual production, due to the accompanying formation of lithium metasilicate and lithium disilicate, uncontrollable formation of lithium disilicate can lead to an increase in the strength of the glass ceramic, which often causes the bur to break and affect grinding efficiency, thereby increasing processing costs. In order to avoid this situation, manufacturers in the industry often produce the lithium metasilicate glass ceramic with low crystallinity by decreasing the crystallization temperature. Although the glass ceramic with low crystallinity can reduce the strength and facilitate grinding, their products have poor toughness due to the high proportion of glass phase, and their edges are prone to cracking when veneers or crowns is processed. In order to solve this problem, some manufacturers in the industry increase the zirconium content in the matrix glass to improve the toughness. However, a small zirconium content is difficult to enhance the toughness. Although a higher zirconium content (mass ratio > 10%) can enhance the toughness and improve grinding performance, its requirements for glass melting are too high, and the costs is high. On the other hand, zirconia has a high density and is difficult to melt, which often leads to zirconium deposition and affects the uniformity of the product. Therefore, there is an urgent need for a low-cost glass ceramic that is not prone to edge cracking during grinding.

**SUMMARY**

In order to overcome the above-mentioned shortcomings of the related art, the present application provides a glass ceramic composition, a preparation method and an application, which solves the technical problem of edge cracking during grinding in glass ceramic products. The specific technical solutions are as follows:

a glass ceramic composition, calculated by mass percentage, including:

58% to 72% of $SiO_2$; 0% to 4% of $Al_2O_3$; 0% to 5% of $Na_2O$; 3% to 8% of $K_2O$; 8% to 17% of $Li_2O$; 2.5% to 5% of $P_2O_5$, 0% to 2% of $MgO$, 0% to 2.5% of $B_2O_3$; 0% to 5% of $ZnO$; and 0% to 3% of $ZrO_2$.

In the glass ceramic composition of the present application, $SiO_2$ is the main network forming agent, which can stabilize the network structure, constitute the main structure of the glass ceramic, and is also the main component of the crystal phase. In the glass ceramic composition, if the content of $SiO_2$ is too low, it can lead to changes in the types of crystal phases, and also weaken the overall performance of the glass ceramic. The content of $SiO_2$ should not be lower than 58 wt%. However, a higher content of $SiO_2$ can lead to difficulty in melting and forming, and the crystal phase can also form a solid solution of $SiO_2$. Therefore, based on comprehensive consideration, the present application controls the content of $SiO_2$ to be 58wt% to 72wt%.

In some embodiments, $Al_2O_3$ is contained, which has a certain effect on the permeability adjustment of the glass ceramic. The higher the content, the better the permeability. However, $Al_2O_3$ is an extremely refractory oxide, which can quickly increase the high-temperature viscosity of the glass and make it more difficult to clarify and homogenize the glass. The bubble defects are not easy to discharge, therefore the content of $Al_2O_3$ is controlled to be less than 4wt%.

The glass ceramic precursor contains alkali metal oxides $R_2O$, which are mainly $Na_2O$, $K_2O$ and $Li_2O$; wherein $Na_2O$ is mainly used to reduce the viscosity of the glass ceramic melt and promote the melting and clarification of the precursor. However, excessive content of $Na_2O$ can increase coefficient of thermal expansion (CTE) and lead to changes in mechanical properties. Therefore, the content of $Na_2O$ is controlled to be 0wt% to 5wt%.

$K_2O$ is beneficial to enhance the gloss of the glass ceramic, and its content is not less than 3%. Not limited to theory, it is found through experiments in the present application that a higher content of $K_2O$ can inhibit the formation of lithium disilicate during secondary sintering, which is not conducive to the final conversion of lithium metasilicate, resulting in a decrease in the performance of dental products. Therefore, the content of $K_2O$ is controlled to be 3wt% to

8wt%.

$Li_2O$ is the main component of the crystal phase. When the content of $Li_2O$ is too low, the crystal phase cannot be formed, and the minimum content needs to be greater than 8wt%. However, too high content of $Li_2O$ can cause uncontrolled crystallization, leading to glass crystallization during molding, thereby resulting in defects and affecting subsequent processes.

$P_2O_5$ can reduce the activated energy of nucleation, facilitate the crystallization of glass, and serve as a nucleating agent for the glass ceramic. If the content of $P_2O_5$ is too low, the glass is difficult to crystallize. The content of $P_2O_5$ is at least 2.5 wt%. However, if the content of $P_2O_5$ is too high, the phase separation of the glass can be severe, which can affect the permeability of the glass ceramic. Therefore, the content of $P_2O_5$ is at most 5 wt%.

In some embodiments, alkaline earth metal oxide RO is contained, and $R^{2+}$ is $Mg^{2+}$ and $Zn^{2+}$, which can improve the chemical stability and mechanical strength of the glass. In the present application, the content of MgO is limited to be 0% to 2wt% and ZnO to be 0% to 5wt%.

$B_2O_3$ is a network former oxide, which can reduce the high-temperature melting viscosity of the glass, improve the melting characteristics, and facilitate the homogenization of glass components at high temperature. However, a high content of $B_2O_3$ can easily lead to phase separation and defects in the glass. Therefore, the content of $B_2O_3$ is controlled to be 0% to 2.5wt%.

In an embodiment, the mass percentage of each component in the glass ceramic composition satisfies the following relationship:

$30\% < (SiO_2 + Al_2O_3 + ZrO_2) - R_2O < 60\%$, where $R_2O$ is a sum of the mass percentage of $Na_2O$, $K_2O$ and $Li_2O$;

$$2\% < SiO_2/(Li_2O+P_2O_5) < 6\%;$$

$$7\% < Li_2O-P_2O_5 < 13.5\%;$$

and

$$0.2\% < (ZrO_2+ZnO+Na_2O+Al_2O_3)/Li_2O < 0.8\%.$$

In an embodiment, when $(SiO_2 + Al_2O_3 + ZrO_2) - R_2O$ is lower than 30%, the overall viscosity of the glass body is low, which provides an extremely convenient flow environment for the movement of crystalline protons, making it difficult to control the crystallization process, affecting the uniformity of crystallization, and also making it easier to convert lithium metasilicate into lithium disilicate. When $(SiO_2 + Al_2O_3 + ZrO_2) - R_2O$ is greater than 60%, the overall viscosity becomes larger, and the overall temperature required the crystalline material rises. In order to match the rapid sintering furnace of a denture factory or hospital, it is necessary to ensure that the secondary sintering temperature is controlled below 900°C. Therefore, $(SiO_2 + Al_2O_3 + ZrO_2) - R_2O$ is controlled to be 30wt% to 60wt%.

In an embodiment, in order to maximize the control of the crystallinity of lithium metasilicate, it is necessary to ensure that the ratio of the mass of $SiO_2$ to the total mass of $Li_2O$ and $P_2O_5$ is greater than 2; when the ratio is too low, excessive $Li_2O$ and $P_2O_5$ in the composition can induce the direct generation of lithium disilicate, resulting in a small proportion of the crystal phase of lithium metasilicate; when the ratio is too high, the excessive $SiO_2$ can form a solid solution of $SiO_2$ during the secondary sintering (T>800°C), resulting in an increase in the crystal phase of the final product and affecting the final performance.

Similarly, in order to avoid the formation of $Li_3PO_4$ crystal phase during the secondary sintering, resulting in an increase in the crystal phase of the final product, $Li_2O-P_2O_5$ is limited to be 7% to 13.5%.

Since dental products have requirements on the final transmittance of the teeth, a higher transmittance appears too bright and unreal, while a too low transmittance appears burnt and lack luster. For this reason, the ratio of the total mass of $ZrO_2$, ZnO, $Na_2O$ and $Al_2O_3$ to the mass of $Li_2O$ is limited to be 0.2% to 0.8%.

In an embodiment, in order to make the color of the glass ceramic product similar to that of natural teeth, the glass ceramic composition further includes colorants and/or fluorescent agents, the content of which is 1% to 6%.

In an embodiment, the colorants and/or fluorescent agents are one or more of $TiO_2$, $La_2O_3$, $V_2O_5$, $CeO_2$, $Er_2O_3$, $Y_2O_3$, $Nd_2O_3$, $Pr_2O_3$, $Mn_2O_3$, NiO and $Fe_2O_3$.

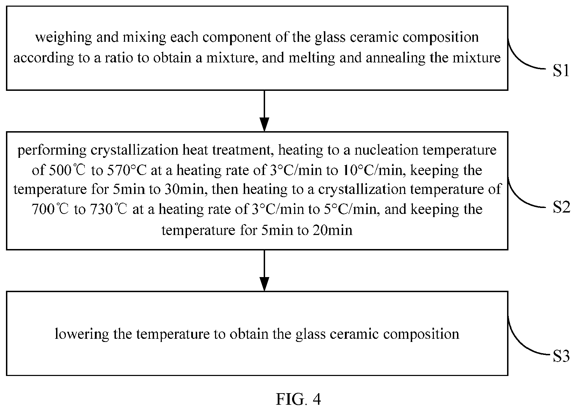

The present application also provides a preparation method of a glass ceramic composition, as shown in FIG. 4, including the following steps:

S1, weighing and mixing each component of the glass ceramic composition described above according to a ratio to obtain a mixture, and melting and annealing the mixture;

S2, performing crystallization heat treatment, heating to a nucleation temperature of 500°C to 570°C at a heating rate of 3°C/min to 10°C/min, keeping the temperature for 5min to 30min, then heating to a crystallization temperature of 700°C to 730°C at a heating rate of 3°C/min to 5°C/min, and keeping the temperature for 5min to 20min; and

S3, lowering the temperature to obtain the glass ceramic composition.

In an embodiment, the melting in step S 1 includes: putting the mixture into a crucible, and melting the mixture at a temperature range of 1400°C to 1500°C for 2h to 4h.

The present application also provides a glass ceramic product prepared according to the above preparation method.

In an embodiment, the crystal phase of the glass ceramic product is lithium metasilicate, and the crystallinity of the glass ceramic composition is greater than 50%.

In an embodiment, the crystal grains of the glass ceramic product are granular, and the average grain size of the crystal grains is 30nm to 60 nm.

After the glass ceramic product is ground, it can be kept at 830°C to 850°C for 5min to 10min in a rapid sintering furnace to complete the sintering of the denture.

After secondary sintering, the flexural strength of the glass ceramic product is greater than 350Mpa.

After secondary sintering, the fracture toughness of the glass ceramic product is greater than $2.2 Mpa.m^{1/2}$.

After secondary sintering, the crystal phase of the glass ceramic product has plate-shaped interlocked lithium disilicate, with a crystallinity of greater than 80%.

The present application also provides an application of the above-mentioned glass ceramic composition or the glass ceramic product prepared according to the above-mentioned preparation method in dental materials, such as the preparation of dentures, veneers, inlays, crown repairs, etc.

Compared with the related art, the present application has the following advantages:

by optimizing the ratio of the glass ceramic composition and combining with a matching process system, the present application eliminates the need to introduce a high proportion of zirconium oxide content and controls the generation of lithium disilicate to increase the crystallinity of the lithium metasilicate glass ceramic, thereby improving the grinding properties of the glass ceramic composition. The present application can prepare lithium metasilicate glass ceramic with a crystal phase of lithium metasilicate, a crystallinity of greater than 50%, a grain size of 30nm to 60nm, and a hardness of $500 kgf/mm^2$ to $590 kgf/mm^2$. The lithium metasilicate glass ceramic does not require the introduction of a high ratio of zirconia content and can be ground to a thickness of 0.2mm without edge collapse. When the glass ceramic is kept at 830°C to 850°C for 5min to 10 min in a rapid sintering furnace, it is possible to prepare the glass ceramic denture product with the crystal phase as lithium disilicate, a crystallinity of greater than 80%, flexural strength of greater than 350MPa and fracture toughness of greater than $2.2 Mpa.m^{1/2}$. The product of the present application has a short holding time for heat treatment, high grinding efficiency, less damage to the bur, and less edge collapse of the product. It has a very competitive advantage and has a great application prospect.

## BRIEF DESCRIPTION OF THE DRAWINGS

In order to more clearly describe the technical solutions in the embodiments of the present application, a brief introduction will be given to the accompanying drawings required in the description of the embodiments. Obviously, the drawings in the following description are only some embodiments of the present application. For those skilled in the art, other drawings can also be obtained based on these drawings without any creative effort.

FIG. 1 is a scanning electron microscope (SEM) image of a sample before secondary sintering according to Embodiment 2 of the present application.

FIG. 2 is a grinding effect picture of the sample before secondary sintering according to Embodiments 1 to 4 of the present application.

FIG. 3 is a SEM picture of the sample after being heated at 840°C for 5 min in a rapid sintering furnace according to Embodiment 3 of the present application.

FIG. 4 is a flowchart showing a preparation method for a glass ceramic composition according to the present application.

## DETAILED DESCRIPTION OF THE EMBODIMENTS

The technical solutions in the embodiments of the present application will be clearly and completely described below with reference to the accompanying drawings in the embodiments of the present application. It is obvious that the embodiments to be described are only some rather than all of the embodiments of the present application. All other embodiments obtained by persons skilled in the art based on the embodiments of the present application without creative efforts shall fall within the scope of the present application.

Performance testing methods and standards:

(1).average grain size: measured using a scanning electron microscope (SEM), scanning a surface by the SEM to observe a diameter of particles, adding up the average diameter size of all grain cross-sections and divided by the number of grains in the SEM image.
(2).crystal phase content: comparing XRD diffraction peaks with the database spectrum to determine the crystal phase, and calculating the proportion of diffraction intensityof the crystal phase in the overall spectrum intensity by a Rietveld method to obtain the crystallinity and amorphous content.
(3).Vickers hardness: GB/T 16534-2009 "Test Method for Room Temperature Hardness of Fine Ceramics", measured using a Vickers hardness tester, with a loading force of 200g and a loading time of 10s.
(4).grinding effect: using an intelligent tooth carving machine.
(5).flexural strength: GB 30367-2013 "Dental Ceramic Materials", tested using a universal testing machine.
(6).fracture toughness: GB 30367-2013 "Dental Ceramic Materials", using a universal testing machine and four-point bending test.

Embodiments 1 to 8 are prepared by the following method: first, weighing each component according to a ratio in Table 1 and mixing evenly, putting the uniform mixture into a crucible made of platinum or platinum rhodium, melting within a temperature range of 1450°C in an electric furnace for 3 h, stirring three times to make it uniform, lowering to an appropriate temperature and casting into a mold, placing the cast glass block into an annealing furnace at 450°C for annealing, and cooling to a room temperature in the furnace and taking out after the annealing. The glass product after the annealing is placed into a crystallization furnace for crystallization heat treatment to obtain the glass ceramic product. The process conditions of the crystallization heat treatment are shown in Table 2. The test results are shown in Table 3.

Table 1. sample composition in Embodiments 1 to 8

| Composition (wt%) | Embodiment 1 | Embodiment 2 | Embodiment 3 | Embodiment 4 | Embodiment 5 | Embodiment 6 | Embodiment 7 | Embodiment 8 |
|---|---|---|---|---|---|---|---|---|
| $SiO_2$ | 68.00 | 59.00 | 70.00 | 71.20 | 67.20 | 62.56 | 69.77 | 67.75 |
| $Al_2O_3$ | 2.05 | 2.90 | 0.00 | 3.20 | 1.92 | 3.78 | 1.56 | 2.25 |
| $Na_2O$ | 0.80 | 2.20 | 3.80 | 0.50 | 3.20 | 2.54 | 0.00 | 0.30 |
| $K_2O$ | 3.60 | 5.80 | 3.50 | 6.48 | 4.80 | 3.10 | 4.41 | 3.70 |
| $Li_2O$ | 12.30 | 15.25 | 13.78 | 10.25 | 14.15 | 15.23 | 16.20 | 15.50 |
| $P_2O_5$ | 3.00 | 4.55 | 3.15 | 2.80 | 4.50 | 3.70 | 3.10 | 3.00 |
| $ZrO_2$ | 1.22 | 2.10 | 0.23 | 0.00 | 1.10 | 0.85 | 0.90 | 2.50 |
| $B_2O_3$ | 0.50 | 0.50 | 1.00 | 0.00 | 0.20 | 0.00 | 0.20 | 0.20 |
| $MgO$ | 0.20 | 0.10 | 0.37 | 0.00 | 0.18 | 0.68 | 0.00 | 0.00 |
| $ZnO$ | 2.88 | 3.33 | 2.05 | 2.11 | 1.74 | 4.50 | 1.85 | 2.25 |
| $TiO_2$ | 0.70 | 0.00 | 0.00 | 0.30 | 0.00 | 0.45 | 0.00 | 0.50 |
| $V_2O_5$ | 1.70 | 2.00 | 0.50 | 0.80 | 0.30 | 0.15 | 0.30 | 0.25 |
| $CeO_2$ | 2.77 | 1.50 | 1.35 | 2.10 | 1.85 | 2.20 | 1.25 | 1.55 |
| $Er_2O_3$ | 0.20 | 0.60 | 0.15 | 0.22 | 0.33 | 0.00 | 0.00 | 0.18 |
| $Y_2O_3$ | 0.00 | 0.00 | 0.02 | 0.03 | 0.00 | 0.01 | 0.00 | 0.00 |
| $Nd_2O_3$ | 0.00 | 0.00 | 0.00 | 0.01 | 0.00 | 0.00 | 0.00 | 0.00 |
| $La_2O_3$ | 0.00 | 0.00 | 0.10 | 0.00 | 0.15 | 0.00 | 0.00 | 0.00 |
| $Mn_2O_3$ | 0.00 | 0.05 | 0.00 | 0.00 | 0.00 | 0.00 | 0.05 | 0.05 |
| $NiO$ | 0.00 | 0.02 | 0.00 | 0.00 | 0.01 | 0.00 | 0.00 | 0.00 |
| $Fe_2O_3$ | 0.03 | 0.00 | 0.00 | 0.00 | 0.00 | 0.25 | 0.41 | 0.02 |
| $Pr_2O_3$ | 0.05 | 0.10 | 0.00 | 0.00 | 0.80 | 0.00 | 0.00 | 0.00 |
| $(SiO_2+ Al_2O_3+ ZrO_2) -R_2O$ | 54.57 | 40.75 | 49.15 | 57.17 | 48.07 | 46.32 | 51.62 | 53.00 |
| $SiO_2/(Li_2O+ P_2O_5)$ | 4.44 | 2.98 | 4.13 | 5.46 | 3.60 | 3.30 | 3.62 | 3.66 |

EP 4 375 253 A1

(continued)

| Composition (wt%) | Embodiment 1 | Embodiment 2 | Embodiment 3 | Embodiment 4 | Embodiment 5 | Embodiment 6 | Embodiment 7 | Embodiment 8 |
|---|---|---|---|---|---|---|---|---|
| $Li_2O\text{-}P_2O_5$ | 9.30 | 10.70 | 10.63 | 7.45 | 9.65 | 11.53 | 13.10 | 12.50 |
| content of colorant | 5.45 | 4.27 | 2.12 | 3.46 | 3.44 | 3.06 | 2.01 | 2.55 |
| $(ZrO_2+ZnO+Na_2O+Al_2O_3)/Li_2O$ | 0.57 | 0.69 | 0.44 | 0.57 | 0.56 | 0.77 | 0.27 | 0.47 |

Table 2. heat treatment process conditions in Embodiment 1 to 8

| heat treatment process | Embodiment 1 | Embodiment 2 | Embodiment 3 | Embodiment 4 | Embodiment 5 | Embodiment 6 | Embodiment 7 | Embodiment 8 |
|---|---|---|---|---|---|---|---|---|
| nucleation temperature (°C) | 520 | 550 | 560 | 530 | 510 | 520 | 550 | 570 |
| nucleation time (min) | 10 | 20 | 20 | 10 | 30 | 25 | 10 | 5 |
| crystallization temperature | 700 | 720 | 720 | 710 | 730 | 710 | 730 | 700 |
| (°C) | | | | | | | | |
| crystallization time (min) | 20 | 10 | 10 | 20 | 5 | 10 | 10 | 10 |
| secondary sintering temperature (°C) | 840 | | | | | | | |
| secondary sintering time (min) | 5 | | | | | | | |

EP 4 375 253 A1

Table 3. test results in Embodiment 1 to 8

| test item | Embodiment 1 | Embodiment 2 | Embodiment 3 | Embodiment 4 | Embodiment 5 | Embodiment 6 | Embodiment 7 | Embodiment 8 |
|---|---|---|---|---|---|---|---|---|
| crystal phase | lithium metasilicate | | | | | | | |
| crystallinity | > 50% | | | | | | | |
| grain size (nm) | 42 | 48 | 45 | 50 | 50 | 46 | 55 | 32 |
| Vickers hardness (kgf/mm$^2$) | 510 | 522 | 514 | 578 | 565 | 570 | 582 | 550 |
| grinding effect (mm) | 0.2, no edge collapse | | | | | | | |
| flexural strength after secondary sintering (MPa) | 355 | 380 | 365 | 408 | 378 | 369 | 388 | 393 |
| fracture toughness after secondary sintering (2Mpa.m$^{1/2}$) | 2.3 | 2.4 | 2.5 | 2.8 | 2.6 | 2.5 | 2.7 | 2.8 |
| crystallinity after secondary sintering | >80% | | | | | | | |

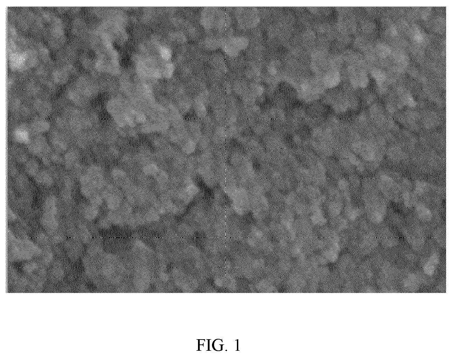

FIG. 1 is a SEM image of the sample before secondary sintering according to Embodiment 2, with the average grain size of 30nm to 60nm.

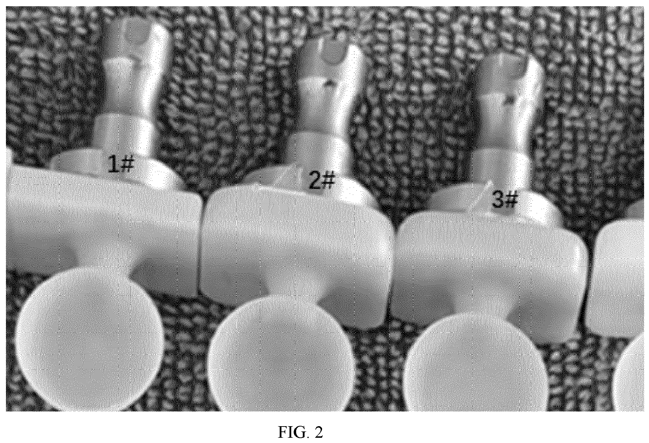

1# to 4# in FIG. 2 show the grinding effect of samples before secondary sintering according to Embodiments 1 to 4, with a thickness of an edge wall of 0.2mm.

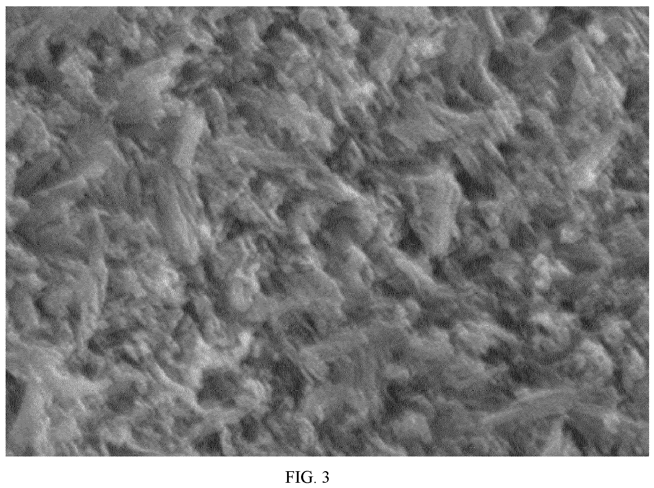

FIG. 3 is a SEM image of the sample after being kept in a rapid sintering furnace at 840°C for 5 min according to Example 3, with a crystal form of plate-like.

Comparative example 1

The difference between Comparative Example 1 and Embodiment 1 is only in the nucleation temperature. The nucleation temperature in Comparative Example 1 is 450°C. It is measured that the crystal phase of the sample in Comparative Example 1 contains very little lithium metasilicate, the crystallinity is 38%, the grain size is 52nm, the Vickers hardness is 446kgf/mm$^2$, teeth appear edge collapse during grinding a thickness of 0.2mm, the flexural strength after the second sintering is 306MPa, the fracture toughness after the second sintering is 1.5Mpa.m$^{1/2}$, and the crystallinity after the second sintering is 58%.

Comparative example 2

The difference between Comparative Example 2 and Embodiment 1 is only in the nucleation temperature. The nucleation temperature of Comparative Example 2 is 600°C. It is measured that the crystal phase of the sample in Comparative Example 2 is lithium metasilicate and lithium disilicate, the crystallinity is 62%, the grain size is 30nm, and the Vickers hardness is 635 kgf/mm$^2$. The hardness is too high, a bur breaks during grinding, which affects the life of the bur. The grinding effect is 0.2mm, the flexural strength after the second sintering is 377MPa, the fracture toughness after the secondary sintering is 2.3Mpa.m$^{1/2}$, and the crystallinity after the secondary sintering is greater than 80%.

Comparative example 3

The difference between Comparative Example 3 and Embodiment 1 is only in the crystallization temperature. The crystallization temperature of Comparative Example 3 is 650°C. It is measured that the crystal phase of the sample in Comparative Example 3 is lithium metasilicate, the crystallinity is 43%, the grain size is 37nm, the Vickers hardness is 487 kgf/mm$^2$, the teeth appear edge collapse during grinding a thickness of 0.2mm,, the flexural strength after the secondary sintering is 343MPa, the fracture toughness after the secondary sintering is 1.95Mpa.m$^{1/2}$, and the crystallization after the secondary sintering is 76%.

Comparative example 4

The difference between Comparative Example 4 and Embodiment 1 is only in the crystallization temperature. The crystallization temperature of Comparative Example 4 is 750°C. It is measured that the crystal phase of the sample in Comparative Example 4 is lithium metasilicate and lithium disilicate, the crystallinity is 72%, the grain size is 65nm, the Vickers hardness is 667 kgf/mm$^2$. The hardness is too high, the bur breaks during grinding to a thickness of 0.2mm, which affects the life of the bur. The flexural strength after the second sintering is 382MPa, the fracture toughness after the second sintering is 1.3Mpa.m$^{1/2}$, and the crystallinity after the secondary sintering is greater than 80%.

The above are only some embodiments of the present application and are not intended to limit the present application. Any modifications, equivalent substitutions and improvements made within the spirit and principles of the present application shall be included in the scope of the present application.

**Claims**

1. A glass ceramic composition, calculated by mass percentage, **characterized by** comprising:
58% to 72% of $SiO_2$; 0% to 4% of $Al_2O_3$; 0% to 5% of $Na_2O$; 3% to 8% of $K_2O$; 8% to 17% of $Li_2O$; 2.5% to 5% of $P_2O_5$, 0% to 2% of MgO, 0% to 2.5% of $B_2O_3$; 0% to 5% of ZnO; and 0% to 3% of $ZrO_2$.

2. The glass ceramic composition according to claim 1, wherein the mass percentage of each component in the glass ceramic composition satisfies the following relationship:
30%< ($SiO_2$+ $Al_2O_3$+ $ZrO_2$)-$R_2O$<60%, where $R_2O$ is a sum of the mass percentage of $Na_2O$, $K_2O$ and $Li_2O$.

3. The glass ceramic composition according to claim 1, wherein the mass percentage of each component in the glass ceramic composition satisfies the following relationship:
$2\% < SiO_2/(Li_2O+P_2O_5) < 6\%$.

4. The glass ceramic composition according to claim 1, wherein the mass percentage of each component in the glass ceramic composition satisfies the following relationship:
$7\% < Li_2O-P_2O_5 < 13.5\%$.

5. The glass ceramic composition according to claim 1, wherein the mass percentage of each component in the glass ceramic composition satisfies the following relationship:
$0.2\% < (ZrO_2+ZnO+Na_2O+Al_2O_3)/Li_2O < 0.8\%$.

6. The glass ceramic composition according to claim 1, further comprising 1wt% to 6wt% colorant and/or fluorescent agent.

7. The glass ceramic composition according to claim 1, wherein a crystal phase of the glass ceramic composition is lithium metasilicate, and a crystallinity of the glass ceramic composition is greater than 50%.

8. A preparation method of a glass ceramic composition, **characterized by** comprising the following steps:

   (S 1), weighing and mixing each component according to a ratio of the glass ceramic composition described in any one of claims 1 to 7 to obtain a mixture, and melting and annealing the mixture;
   (S2), performing crystallization heat treatment, heating to a nucleation temperature of 500°C to 570°C at a heating rate of 3°C/min to 10°C/min, keeping the temperature for 5min to 30min, then heating to a crystallization temperature of 700°C to 730°C at a heating rate of 3°C/min to 5°C/min, and keeping the temperature for 5min to 20min; and
   (S3), lowering the temperature to obtain the glass-ceramic composition.

9. The preparation method of the glass ceramic composition according to claim 8, wherein melting the mixture in S1 comprises: putting the mixture into a crucible, and melting the mixture at a temperature range of 1400°C to 1500°C for 2h to 4h.

10. An application of the glass ceramic composition according to any one of claims 1 to 7 in dental materials.

FIG. 1

FIG. 2

FIG. 3

weighing and mixing each component of the glass ceramic composition according to a ratio to obtain a mixture, and melting and annealing the mixture — S1

performing crystallization heat treatment, heating to a nucleation temperature of 500℃ to 570°C at a heating rate of 3°C/min to 10°C/min, keeping the temperature for 5min to 30min, then heating to a crystallization temperature of 700℃ to 730℃ at a heating rate of 3°C/min to 5°C/min, and keeping the temperature for 5min to 20min — S2

lowering the temperature to obtain the glass ceramic composition — S3

FIG. 4

Europäisches
Patentamt

European
Patent Office

Office européen
des brevets

# EUROPEAN SEARCH REPORT

Application Number

EP 23 20 0245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/236971 A1 (RAMPF MARKUS [CH] ET AL) 18 August 2016 (2016-08-18) * the whole document * * in particular example 5 and [0063] * | 1-10 | INV. C03C3/097 C03C4/00 C03C10/00 |
| X | US 2020/317561 A1 (ARNOLD LARS [CH] ET AL) 8 October 2020 (2020-10-08) * the whole document * * in particular examples 1, 3, 6-7 and 9 * | 1-10 | |
| X | DE 10 2005 028637 A1 (IVOCLAR VIVADENT AG [LI]) 21 December 2006 (2006-12-21) * the whole document * * in particular examples 2-8 * | 1-10 | |
| X | CN 113 264 684 A (SHENZHEN UPCERA BIOLOGICAL MAT CO LTD) 17 August 2021 (2021-08-17) * paragraphs [0001] - [0011]; claims; examples 1,3-5 * | 1-6,10 | |
| X | US 10 131 569 B2 (IVOCLAR VIVADENT AG [LI]) 20 November 2018 (2018-11-20) * the whole document * * in particular example 12 and column 5 (lines 38-46) * | 1-10 | **TECHNICAL FIELDS SEARCHED (IPC)** C03C |

The present search report has been drawn up for all claims

| Place of search | Date of completion of the search | Examiner |
|---|---|---|
| Munich | 18 April 2024 | Heer, Stephan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 20 0245**

This annex lists the patent family members relating to the patent documents cited in the above-mentioned European search report.
The members are as contained in the European Patent Office EDP file on
The European Patent Office is in no way liable for these particulars which are merely given for the purpose of information.

**18-04-2024**

| Patent document cited in search report | | | Publication date | Patent family member(s) | | | Publication date |
|---|---|---|---|---|---|---|---|
| US 2016236971 | A1 | | 18-08-2016 | CA | 2926665 | A1 | 14-05-2015 |
| | | | | CN | 105683110 | A | 15-06-2016 |
| | | | | CN | 112645601 | A | 13-04-2021 |
| | | | | EP | 2868634 | A1 | 06-05-2015 |
| | | | | ES | 2891275 | T3 | 26-01-2022 |
| | | | | JP | 6571076 | B2 | 04-09-2019 |
| | | | | JP | 2017501098 | A | 12-01-2017 |
| | | | | KR | 20160078995 | A | 05-07-2016 |
| | | | | US | 2016236971 | A1 | 18-08-2016 |
| | | | | WO | 2015067643 | A1 | 14-05-2015 |
| US 2020317561 | A1 | | 08-10-2020 | CN | 111792847 | A | 20-10-2020 |
| | | | | EP | 3718980 | A1 | 07-10-2020 |
| | | | | ES | 2926479 | T3 | 26-10-2022 |
| | | | | JP | 2020169118 | A | 15-10-2020 |
| | | | | KR | 20200118375 | A | 15-10-2020 |
| | | | | US | 2020317561 | A1 | 08-10-2020 |
| DE 102005028637 | A1 | | 21-12-2006 | NONE | | | |
| CN 113264684 | A | | 17-08-2021 | CN | 113264684 | A | 17-08-2021 |
| | | | | WO | 2022257624 | A1 | 15-12-2022 |
| US 10131569 | B2 | | 20-11-2018 | CA | 2944409 | A1 | 19-11-2015 |
| | | | | CN | 106232542 | A | 14-12-2016 |
| | | | | EP | 2944619 | A1 | 18-11-2015 |
| | | | | ES | 2954898 | T3 | 27-11-2023 |
| | | | | HK | 1211567 | A1 | 27-05-2016 |
| | | | | JP | 6700199 | B2 | 27-05-2020 |
| | | | | JP | 2017520498 | A | 27-07-2017 |
| | | | | KR | 20170008746 | A | 24-01-2017 |
| | | | | US | 2017144919 | A1 | 25-05-2017 |
| | | | | US | 2019039944 | A1 | 07-02-2019 |
| | | | | WO | 2015173230 | A1 | 19-11-2015 |

EPO FORM P0459

For more details about this annex : see Official Journal of the European Patent Office, No. 12/82